# EUROPEAN PATENT SPECIFICATION

(11) **EP 3 996 591 B1**
(45) Date of publication and mention of the grant of the patent: **15.01.2025**
(21) Application number: 20840177.8
(22) Date of filing: 17.06.2020
(51) Int. Cl.: A61B 5/1468, A61B 5/15, A61B 5/145

(54) **INTEGRATED SENSOR ASSEMBLY UNIT FOR SAMPLING OF A BODILY FLUID**
INTEGRIERTE SENSOREINHEIT ZUR PROBENENTNAHME EINER KÖRPERFLUSSIGKEIT
UNITÉ AVEC CAPTEUR INTÉGRÉ POUR LE PRÉLÈVEMENT D'UN LIQUIDE CORPOREL

(30) Priority: 12.07.2019 SE 1950886
(43) Date of publication of application: 18.05.2022
(73) Proprietor: Ascilion AB, 164 25 Kista (SE)
(72) Inventor: RANGSTEN, Pelle, 743 40 Storvreta (SE); RENLUND, Markus, 184 60 Åkersberga (SE)
(74) Representative: Zacco Sweden AB
(86) International application number: PCT/SE2020/050633
(87) International publication number: WO 2021/010879

(56) References cited:
- WO-A1-2016/164208
- WO-A1-2017/095321
- WO-A1-2019/020327
- US-A1- 2002 169 394
- US-A1- 2005 209 565
- US-A1- 2016 058 342
- US-A1- 2017 347 925
- US-A1- 2018 338 713
- US-B1- 8 506 529
- No further relevant documents disclosed

## Description

### TECHNICAL FIELD

The present invention relates in general to a sensor assembly for sampling of a bodily fluid. The present invention relates in particular to a sensor assembly provided on a first substrate having at least one capillary bore and a second substrate having two metallised vias and a sensor. The present invention also relates to a sensor assembly where the first substrate is having a plurality of microneedles where the capillary bores also extend through the plurality of microneedles.

### BACKGROUND ART

There are numerous ways to sample bodily fluids, for example by using a syringe. This is cumbersome and improved alternatives exist, such as by using microneedles. Although many application fields exist for microneedles, the vast majority of published microneedles concern drug delivery in various forms.

For example the concept of an array of miniaturized needles for drug delivery purposes dates back to the 70's US 3,964,482. One of the earliest reported microneedles in the scientific literature was an out-of-plane silicon needle array featuring 100, 1.5 mm long, needles on an area of 4.2 mm×4.2 mm in "A silicon-based, three-dimensional neural interface: manufacturing processes for an intercortical electrode array.", IEEE Trans Biomed Eng. 1991 Aug; 38(8):758-68, Campbell et al. Eventually bio sensing technology will be to the 21st century what microelectronics was to the second half of the 20th century.

Integrated circuits (IC) have had an enormous impact on our daily life today and making use of the same miniaturization and cost benefits of volume manufacturing bio sensing might move clinical diagnosis and health monitoring from expensive laboratories to small hand-held consumer devices. Sampling of an analyte to be measured is a prerequisite for bio sensing. Many of the designs described in scientific papers have the purpose of extracting bodily fluids. For example blood or interstitial fluid, ISF. Different bodily fluids demand a variety of solutions, for example successful extraction of blood has been demonstrated with use of the natural "overpressure" in the vascular system, while successful extraction of ISF without under-pressure, through diffusion or other mechanisms such as capillary forces are rare or even non-existing. The terms "under-pressure" and "sub-pressure" are used as equivalents in the present disclosure

A previously known document US2016/058342A1 discloses a sensor assembly for sampling of a bodily fluid comprising two substrates. One of the substrate comprises micro needles for piercing the stratum corneum and sampling interstitial fluid by capillary action. The second substrate supports micro sensors, a control circuitry and a reference electrode. Perforations in the second substrate are used to vent and to incorporate electrical conductors and provide electrical access to the sensors and control circuitry.

Independent of method of sampling, the sample has to be transferred to the sensing device in a controlled manner. In order to further improve usage and prevent mistakes, this can preferably be performed in an integrated unit.

### SUMMARY OF THE INVENTION

The aim of the present invention is to set aside the abovementioned drawbacks and shortcomings of the previously known sensor assemblies and to provide an improved solution for sampling of bodily fluids.

In order to improve the extraction of fluids such as ISF an under-pressure can be applied. This is however difficult to combine with collecting readings from an integrated sensor. It has been realized that a fluid path extending through the sensor assembly is beneficial. In addition, in order to have an operational sensor the comprised electrodes need to be in communicating connection with an exterior part of the sensor assembly.

An object of the invention is to provide an improved sensor assembly having a fluid path extending through the assembly and integrated operational sensor that allows easy sampling of bodily fluid, such as interstitial fluid, ISF.

The object of the invention is met in a sensor assembly as defined in the appending claims.

In a first aspect, the present invention relates to a sensor assembly for sampling of a bodily fluid comprising a first substrate, a second substrate and a sensor. The first substrate comprises at least one capillary bore defining a fluid path. The fluid path is extending through said first substrate from a top side to a fluid channel on a bottom side of said first substrate. The second substrate is arranged in connection with the first substrate and the fluid channel of the first substrate is in fluid communication with a first metallised via and a second metallised via formed in the second substrate. Thereby extending the fluid path through the first metallised via and the second metallised via. The sensor comprises a first electrode and a second electrode. The first electrode and the second electrode is arranged on the second substrate in fluid communication with the fluid channel of the first substrate. The first electrode is in electric contact with the first metallised via and the second electrode is in electric contact with the second metallised via.

In embodiments, the first substrate may comprise a plurality of bores.

In embodiments, the sensor assembly may further comprise means for applying sub-pressure to the fluid path to draw fluid from the capillary bores towards the second substrate.

In embodiments, the sensor assembly may further comprise a plurality of microneedles integrally formed on the first substrate. Each microneedle may comprise an elongated body extending from a distal end thereof to a proximal end thereof on the first substrate along a longitudinal axis. Each microneedle may further have a bevel at the distal end. The capillary bores may extend through the elongated body in a longitudinal direction thereof and further define the fluid path. The proximal end may be integrally formed with the first substrate and the fluid path may be in fluid communication with the fluid channel of the first substrate.

In embodiments, the second substrate may be arranged in connection with the first substrate on a side opposite the plurality of microneedles.

In embodiments, the first electrode may be shaped as a spiral and the second electrode may be shaped as a spiral, and wherein the spiral shapes of the first and second electrodes are nested.

In embodiments, the sensor is located on a side of the second substrate directed towards the first substrate.

In embodiments, the sensor may at least partly be located on the side of the second substrate that is directed towards the first substrate.

In embodiments, the sensor may at least partly be located in the via.

In embodiments, the via may further comprise a signal path that may be extending through the second substrate and the sensor may be arranged in electrical connection with the signal path.

In embodiments, the via may be hollow, thereby providing fluid communication between two opposite sides of the second substrate.

In embodiments, the sensor may be an electrochemical sensor. Electrochemical sensors are known in the art. One known type of electrochemical glucose sensor is the Clark biosensor. This sensor is based on a thin layer of glucose oxidase (GOx) on an oxygen electrode. The readout is the amount of oxygen consumed by GOx during enzymatic reaction with the substrate glucose. A more detailed description of biosensors, such as the Clark type, can be found in Anthony P.F. Turner: Biosensors: sense and sensibility, Chem. Soc. Rev., Volume 42, Number 8, 21 April 2013, pages 3175-3648. The described sensors may be adapted for use in the present invention. In such embodiments, at least one electrode of the sensor is coated with an enzyme, such as a redox enzyme. Enzymes useful in the present invention are oxidoreductases acting on an electron donor with oxygen as acceptor. Such enzymes are generally classified in the group EC 1.X.X.X in the enzyme nomenclature of the International Union of Biochemistry and Molecular Biology. One preferred enzyme is glucose oxidase (EC 1.1.3.4).

In embodiments, the sides of the via may further comprise a substance with a specified surface energy. By this, the flow behaviour of the fluid may be controlled by its interaction with the specified surface energy.

In embodiments, the wall surface of the via may further be made hydrophobic, such as by coating with a hydrophobic substance.

In embodiments, the wall surface of the via may further be made hydrophilic, such as by coating with a hydrophilic substance.

In embodiments, a cross-sectional area of the capillary bore in the distal end may be larger than the cross-sectional area of the capillary bore in the proximal end.

In embodiments, the fluid channel may further comprise a decreasing cross-sectional area in order to further enhance a fluid flow in the fluid channel.

The term cross-sectional area means the area of a cross section of an object. The cross section is the intersection of the object and a plane. In comparing different cross-sectional areas it is understood that the intersecting planes are parallel unless otherwise stated.

In embodiments, the first substrate may further comprise a frame located on the same side as the plurality of microneedles, wherein the frame at least partly surrounds an area on the first substrate having the plurality of microneedles.

In embodiments, the frame may have a height in a direction extending from the substrate that is equal or higher than the height of the plurality of microneedles.

In embodiments, the cross sectional area of the capillary bore of the microneedles may gradually decrease from the distal end towards the proximal end along the longitudinal direction. This contributes to an enhanced fluid flow through the capillary bore, by means of capillary force acting on the fluid in the capillary bore.

In embodiments, the cross-section (crosswise to the longitudinal direction) of the capillary bore may further comprise at least one rounded corner. This contributes to the wetting of the capillary bore, which has a positive effect on the fluid flow.

In embodiments, the capillary bore may have a triangular cross-section. A triangular cross-section has been demonstrated to provide a very good fluid flow in the capillary bore. A triangular cross-section within this application encompasses cross sections with substantially triangular shape, i.e. edges with convex or concave shape or straight shape, corners with sharp angles and corners with blunt angles as well as rounded corners.

In embodiments, the walls of the capillary bore may comprise hydrophilic surfaces, which enhances the fluid flow in the capillary bore.

In embodiments, the fluid channel may be configured to provide an under-pressure, relative the atmospheric pressure, to the capillary bore, whereby fluid flow through the capillary bore is enhanced. An under-pressure may for example be created with a syringe connected to the fluid channel.

In embodiments, the second substrate may be operatively connected to the first substrate by means of anodic or direct bonding, which provides a strong and fluid tight seal without the risk of clogging the fluid channels with adhesive.

In a second aspect, the present invention relates to a measurement device comprising a sensor assembly according to the invention, further comprising a suction device arranged in connection with the sensor assembly on a side opposite the one or plurality of microneedles and in fluid communication with the first via and the second via of the second substrate, thereby providing a pressure difference through the vias and the fluid channel.

In embodiments, the one or plurality of microneedles integrally formed on the first substrate may comprise an elongated body extending from a distal end with a bevel to a proximal end on the substrate along a longitudinal axis. The elongated body may comprise a capillary bore extending in a longitudinal direction thereof and that defines a fluid path. The proximal end may be integrally connected with the substrate and the capillary bore may be in fluid communication with a fluid channel of the first substrate. Further, the cross-sectional area of the capillary bore in the distal end may be larger than the cross-sectional area of the capillary bore in the proximal end.

A bevel is referred to as a bevelled surface relative the longitudinal axis of the capillary bore.

### BRIEF DESCRIPTION OF THE DRAWINGS

A more thorough understanding of the abovementioned and other features and advantages of the present invention will be evident from the following detailed description of embodiments with reference to the enclosed drawings, on which:
Figure 1 is a schematic cross section view of a sensor assembly.
Figure 2a is a schematic perspective view of a sensor assembly shown from above with a plurality of microneedles.
Figure 2b is a schematic perspective view of a sensor assembly shown from below.
Figure 3 is a schematic perspective view of a cross section of a sensor assembly with a plurality of microneedles.
Figure 4 is a cross-sectional view of a sensor assembly with a microneedle.
Figure 5a is a cross-sectional view along the longitudinal axis of a microneedle according to an embodiment.
Figure 5b is a cross-sectional view along the longitudinal axis of a microneedle according to an embodiment.
Figure 5c is a cross-sectional view along the longitudinal axis of a microneedle according to an embodiment.
Figure 6 is a bottom view of a chip.
Figure 7 is a perspective top view of the second substrate with vias and a sensor.
Figure 8 is a schematic cross section view of a sensor assembly having multiple bores and a plurality of microneedles.

### DETAILED DESCRIPTION

The present invention is based on the insights disclosed. When examined carefully it's clear that the sensor assemblies in the prior art for sampling of bodily fluids either can't apply under-pressure or be provided in an integrated unit. Solution that are able to apply under-pressure rely on transfer of the sample to the sensing device. Similarly, solutions that are able to provide integrated units are not able to provide under-pressure. Under-pressure which improves or even is demanded for some samplings.

In order to combine the improved extraction of fluids such as ISF by use of an applied under-pressure with the improve usage that an integrated unit provides, an improved sensor assembly is described.

For increased understanding, some figures illustrates the substrates of the sensor assembly as separated, it is understood that substrates illustrated as separated typically is joint. For an example, the substrates may be joint by means of anodic/direct bonding, which provides a strong and fluid tight seal without the risk of clogging the fluid channels with adhesive. Other means of joining the substrates may be envisioned, such as clamping or by an adhesive.

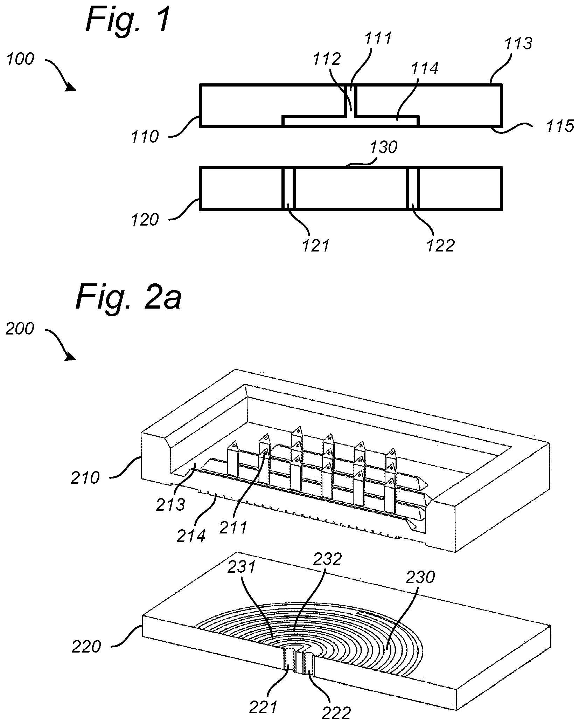

Figure 1 is a schematic cross section view of a sensor assembly 100 according to an embodiment of the present invention having a first substrate 110, a second substrate 120 and a sensor 130.

The first substrate 110 has a capillary bore 111 defining a fluid path 112 extending through said first substrate 110 from a top side 113 to a fluid channel 114 on a bottom side 115 of said first substrate 110.

The second substrate 120 is arranged in connection with the first substrate 110, the fluid channel 114 of the first substrate is in fluid communication with a first metallised via 121 and a second metallised via 122 formed in the second substrate 120, thereby extending the fluid path 112 through the first metallised via 121 and the second metallised via 122.

The sensor 130 comprising a first electrode and a second electrode is arranged on the second substrate 120 in fluid communication with the fluid channel 114 of the first substrate 110. The first electrode is in electric contact with the first metallised via 121 and the second electrode is in electric contact with the second metallised via 122.

The first metallised via 121 and the second metallised via 122 may be hollow, thereby providing fluid communication between two opposing sides of the second substrate 120.

The sides of the vias may further comprise a substance with a specified surface energy. By this, the flow behaviour of the fluid may be controlled by its interaction with the specified surface energy. For an example, the sides of the via may comprise a hydrophobic substance or a hydrophilic substance.

The interior dimensions and geometry of the bore may also be designed to allow capillary forces to act on liquid in part or all the way through the fluid channel of the sensor assembly 100. These capillary forces may be used together with an applied under-pressure.

In addition, the sensor assembly may further comprise means for applying under-pressure to the fluid path to draw fluid from the capillary bores towards the second substrate. An under-pressure may for example be created with a syringe connected to the fluid channel.

Figure 2a is a schematic perspective view of a sensor assembly 200 shown from above. The sensor assembly 200 is having a first substrate 210, a second substrate 220 and a sensor 230.

The first substrate 210 has a capillary bore 211, here shown on a microneedle, defining a fluid path extending through said first substrate 210 from a top side 213 to a fluid channel 214 on a bottom side of said first substrate 210. The first substrate is here shown with a plurality of microneedles. In other embodiments, the first substrate 210 may be without microneedles, the capillary bore 211 may then be located on the top side 213.

The second substrate 220 is illustrated at a distance from the first substrate 210, the fluid channel of the first substrate is in fluid communication with a first metallised via 221 and a second metallised via 222 formed in the second substrate 220, thereby extending the fluid path through the first metallised via 221 and the second metallised via 222.

The sensor 230 comprising a first electrode 231 and a second electrode 232 is arranged on the second substrate 220 in fluid communication with the fluid channel of the first substrate 210. The first electrode 231 is in electric contact with the first metallised via 221 and the second electrode 232 is in electric contact with the second metallised via 222.

The sensor assembly 200 may have one or a plurality of microneedles. The microneedles may be integrally formed on the first substrate 210. Each microneedle may comprise an elongated body extending from a distal end thereof to a proximal end thereof on the top side 213 of the first substrate 210 along a longitudinal axis. Each microneedle may further have a bevel at the distal end. The capillary bores may extend through the elongated body in a longitudinal direction thereof and further define the fluid path. The proximal end may be integrally formed with the first substrate 210 and the fluid path may be in fluid communication with the fluid channel 214 of the first substrate 210.

The sensor 230 is here illustrated on a side of the second substrate 220 that is directed towards the first substrate 210. In other examples, the sensor may comprise an elongated portion extending in the vias in the second substrate 220. For example, the sensor may be a electro chemical sensor.

The vias may each further comprise a signal path that may be extending through the second substrate 220. The sensor 230 may be arranged in electrical connection with the signal paths.

The first electrode 231 may be shaped as a spiral and the second electrode 232may be shaped as a spiral, and wherein the spiral shapes of the first and second electrodes may be nested.

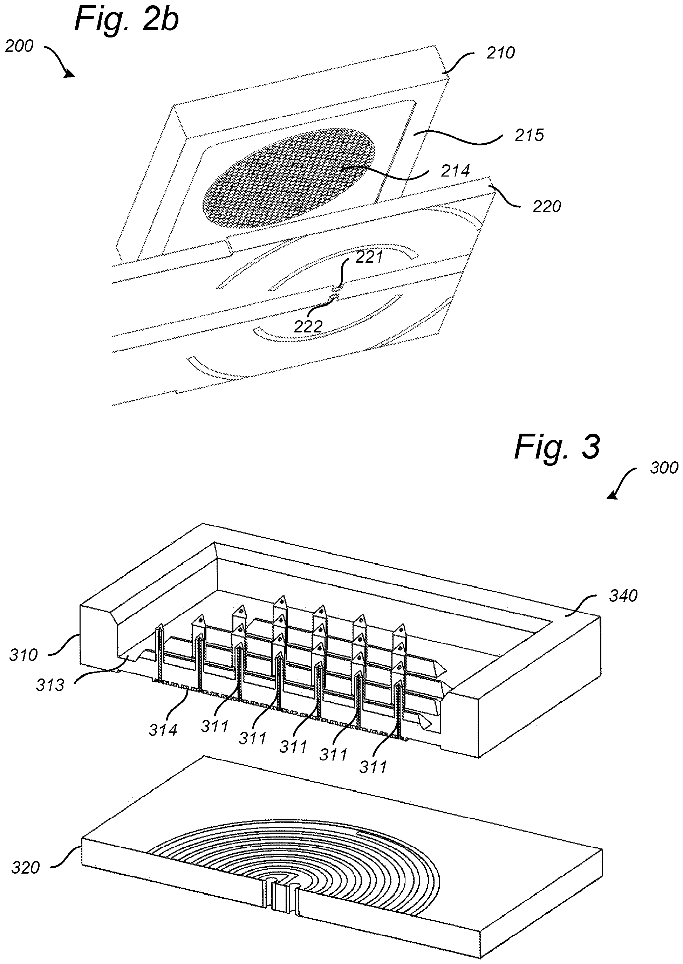

Figure 2b is a schematic perspective view of a sensor assembly 200 shown from below. The sensor assembly 200 is having a first substrate 210, a second substrate 220 and a sensor.

The first substrate 210 has a capillary bore defining a fluid path extending through said first substrate 210 from a top side to a fluid channel 214, here shown as extending over a surface, on a bottom side 215 of said first substrate 210.

The second substrate 220 is illustrated at a distance from the first substrate 210, the fluid channel 214 of the first substrate is in fluid communication with a first metallised via 221 and a second metallised via 222 formed in the second substrate 220, thereby extending the fluid path through the first metallised via 221 and the second metallised via 222.

The sensor comprising a first electrode and a second electrode is arranged on the second substrate 220 in fluid communication with the fluid channel 214 of the first substrate 210. The first electrode is in electric contact with the first metallised via 221 and the second electrode is in electric contact with the second metallised via 222.

The fluid channel 214 on the backside of the first substrate 210 is shown in the figure. At least one fluid channel port on the backside of the first substrate 210 is connected between the fluid path through the first substrate to at least one fluid channel 214, thereby fluidly connecting the at least one capillary bores with the fluid channel 214. The at least one fluid channel 214 may for example be a network of interconnected channels. The fluid channel 214 may have a width that is larger than the depth of the fluid channel 214.

Figure 3 is a schematic perspective view of a cross section of a sensor assembly 300 with a plurality of microneedles showing the fluid path of the sensor assembly. The sensor assembly 300 is having a first substrate 310, a second substrate 320 and a sensor.

The first substrate 310 has a plurality of capillary bores 311, here shown through a plurality of microneedles, defining a fluid path extending through said first substrate 310 from a top side 313 to a fluid channel 314 on a bottom side of said first substrate 310. In the present figure, the cross section of the fluid channel 314 can be seen.

The second substrate 320 and the sensor is arranged according to the previously presented figures.

The sensor assembly may also have a frame structure dimensioned to support the tip of a finger constructed as structure protruding along the longitudinal direction of the microneedles, and preferably having a diameter of less than 15 mm.

The sensor assembly may in some examples be at least partly surrounded by a frame structure dimensioned to support the tip of a finger. Thereby the skin of the tip of the finger may be supported and tensioned to facilitate penetration of the at least one microneedle into the skin.

The sensor assembly may also be protected by a surrounding structure protruding to at least the same plane as the tip of the needles enabling for instance handling of wafers in the case of MEMS manufacturing of the herein described chipset.

The sensor assembly may also have a surrounding structure that stretches the skin prior to penetration by the above mentioned plurality of needles.

An example of a surrounding frame structure 340 on the top side of the first substrate 310 is also illustrated in figure 3.

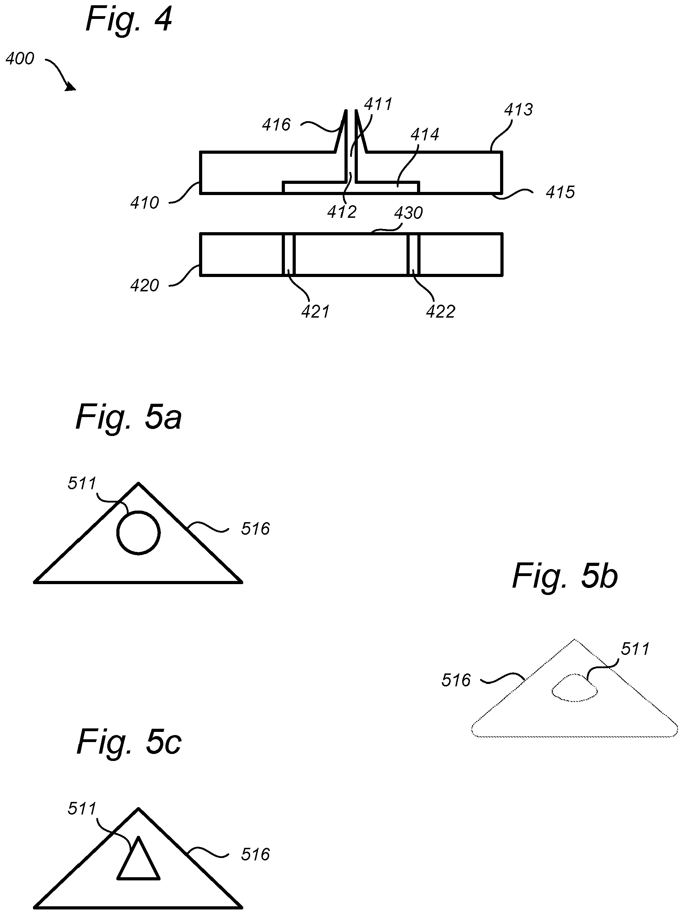

Figure 4 is a cross-sectional view of a schematic sensor assembly 400 with a microneedle 416. The sensor assembly 400 is having a first substrate 410, a second substrate 420 and a sensor 430.

The first substrate 410 has a capillary bore 411 defining a fluid path 412 extending through said first substrate 410 from the microneedle 416 on the top side 413 to a fluid channel 414 on a bottom side 415 of said first substrate 410.

The second substrate 420 and the sensor 430 are arranged according to the previously presented figures. At least by this, the fluid path 412 extends through the microneedle 416, the fluid channel 414 and the metallised vias 421, 422. Thereby a fluid in the fluid path 412 may pass the sensor 430 while passing the first substrate 410 and the second substrate 420.

The microneedle 416 may have a sharp tip defined by crystallographic planes. For example, the microneedle may have a bevel slope that for each needle or needles is defined by the 111 planes.

Each microneedle 416 may comprise a capillary bore 411, e.g. a single capillary bore. Thereby bodily fluid may be extracted by means of capillary suction through the microneedle 416.

The microneedle 416 may be provided with a cap at a distal end for shielding the capillary bore from clogging, whereby at least one opening to the capillary bore is provided in a lateral direction of the microneedle, perpendicular to the axial or longitudinal extension of the microneedle.

The capillary bore of each microneedle may be provided with a hydrophilic surface. Thereby capillary flow of bodily fluid may be assisted.

The microneedle may comprise a plurality of cutting elements extending along a longitudinal direction of the microneedle. Thereby the skin may be cut and opened to facilitate extraction of bodily fluid.

The perimeter of the bore hole in each microneedle as projected on the bevel of the needle may be located at a distance from the tip in a way where the tip is outside the perimeter.

The microneedle 416 may have a length of 200-1000 µm, preferably 400-900 µm, more preferably 300-600 µm, and an outer circumference of 400-800 µm. By this, the microneedle 416 has dimensions suitable for penetration of the skin and extraction of bodily fluid.

A portion of the bore hole as projected on the side that contains the capillary system may be outside the connecting capillary generating a maximized wall surface that minimizes surface tension.

The connection between the bore hole and the capillary may for example be designed with a minimized contact angle, thereby enabling a tension driven flow.

The shaft of each needle may be provided both with or without a hilt.

The vertical bore holes may be filled with material that is selective and specific to certain molecules and thereby creating an integrated extraction and sensing chipset. The filler material could for instance be glucose oxidase and carbon powder and thereby creating a glucose specific extraction and sensing chipset.

A plurality of openings may be provided in a lateral direction, around a circumference of the microneedle 416. The at least one opening may be provided about midways along a longitudinal extension of the microneedle 416. Thereby the extraction of bodily fluid is facilitated and the risk for clogging is further reduced.

The bores may with their hollow structure constitute inlets for sampling of bodily fluid. Thereby bodily fluid, such as interstitial fluid (ISF) may be extracted and introduced into the sensor 430 with minimal discomfort for the patient.

The sensor 430 may have a collecting network of capillaries in different patterns and as an advantage is collection of liquid and storage made without evaporation problems. Hence, the chip can sample and the analysis can also be made ex situ.

The sensor 430 may have an interface between the vertical bore holes and the collecting capillary laterally misaligned to allow liquid to wet the walls and hence by capillary action fill the collecting channels on the backside.

The microneedle 416 may have the bevel oriented in the crystallographic directions or preferred in the same direction.

Figure 5a is a cross-sectional view along the longitudinal axis of a microneedle 516 according to an embodiment. In this figure a circular cross-section of the capillary bore 511 is shown.

Figure 5b is a cross-sectional view along the longitudinal axis of a microneedle 516 according to an embodiment. In this figure an uneven cross-section of the capillary bore 511 is shown. The microneedle 516 shown has two rounded corners. Other designs are also possible, such as one or three rounded corners. The corners may have the same or different radiuses of curvature. In addition, the corners may be cut in such a way that the triangle may be described as having six corners, with three sides that are considerably larger than the other three sides.

Figure 5c is a cross-sectional view along the longitudinal axis of a microneedle 516 according to an embodiment. In this figure a triangular cross-section of the capillary bore 511 is shown.

In this application a triangular cross-section of should encompass a shape with three edges connected with corresponding corners. The edges may be straight, curved, convex or concave.

The corners may be sharp, blunt or rounded with different or the same radius. Thus, within this application a cross-section with the shape of an egg or a heart is considered to be triangular. This reasoning applies both to the shape of the bore as to the shape of the microneedle.

For example a triangular shape of the capillary bore 511 may be substantially triangular with a convex base connected to straight sections via a curved corner. This shape of the capillary bore has been demonstrated to be very efficient for extracting interstitial fluid from a finger of a human test subject.

For example, the cross-sectional area of the capillary bore in the distal end may be larger than the cross-sectional area of the capillary bore in the proximal end. In addition, the fluid channel may further comprise a decreasing cross-sectional area in order to further enhance a fluid flow in the fluid channel. The cross sectional area of the capillary bore of the microneedles may for example gradually decrease from the distal end towards the proximal end along the longitudinal direction.

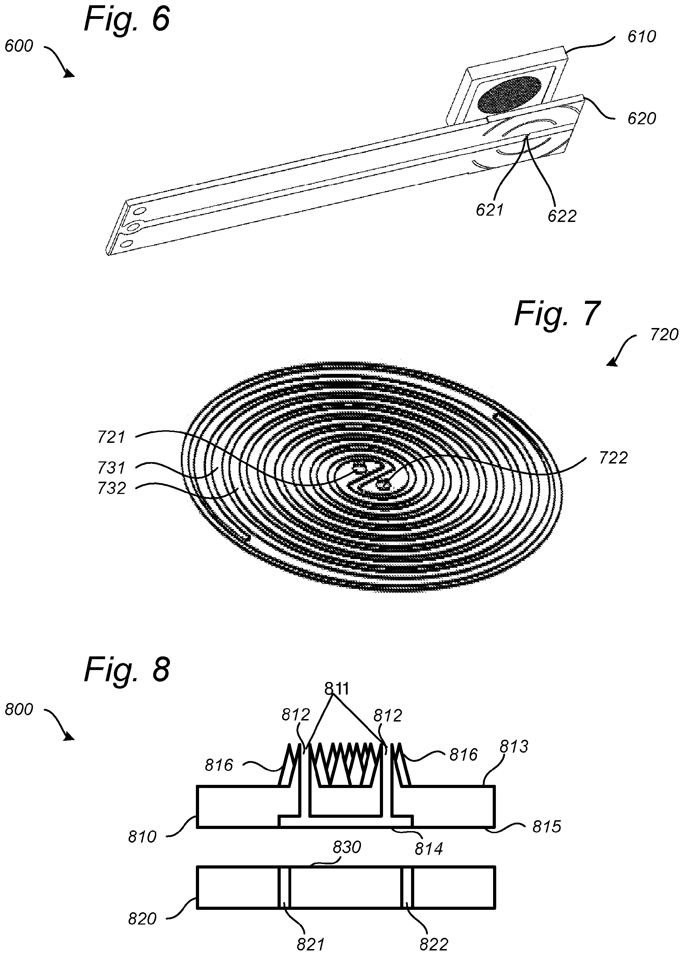

Figure 6 is a schematic bottom view of a chip having a sensor assembly according to an embodiment of the present invention. Shown is a first substrate 610 and a second substrate 620. The second substrate 620 has a first metallised via 621 and a second metallised via 621. The metalized vias may be used to communicate readings from a sensor on the opposing side of the second substrate 620 and is in electric contact with extended pads. The extended pads may be used to further simplify reading from the sensor.

Figure 7 is a top view of the second substrate 720 with vias and a sensor. The view is in perspective, hence there may be some distortion present in the figure.

On the second substrate 720 a first metallised via 721 and a second metallised via 722 is formed. The second substrate further comprise a sensor having a first electrode 731 and a second electrode 732, both can be seen in the figure as spirals. The first electrode 731 is in electric contact with the first metallised via 721 and the second electrode 732 is in electric contact with the second metallised via 722. By this, readings from the electrodes may be communicated through the second substrate 720 by the first metallised via 721 and the second metallised via 722.

The sensor assembly may also be provided with a plurality of needles organized in an array or matrix at a minimum distance from each other of 100 micrometres but not greater than 1mm apart, has a sharp tip in the same plane or slightly below a surrounding structure and where the tip may have a 54.7 degree bevel and the needle a hollow bore with a capillary dimension that allows extraction without clogging and a shaft, longer than 200 micrometres.

Figure 8 is a schematic cross section view of a schematic sensor assembly having multiple bores 812 from a plurality of microneedles 816. The sensor assembly 800 is having a first substrate 810, a second substrate 820.

The first substrate 810 has multiple capillary bores (811) defining a fluid path 812 extending through the first substrate 810 from the plurality of microneedles 816 on the top side 813 to a fluid channel 814 on a bottom side 815 of said first substrate 810. The figure is illustrated with capillary bores in two of the plurality of microneedles 816, it is however understood that there may be capillary bores in several of the plurality of microneedles 816 and that each of the capillary bores may be connected with the fluid channel 814.

Each capillary bore may extend from top side 813 of the first substrate 810 to a fluid channel port on the bottom side 815 of the first substrate 810. Should the sensor assembly be provided with microneedles, the capillary bore may extend from the tip of the microneedles to a fluid channel port on the bottom side 815 of the first substrate 810.

The second substrate 820 comprise a sensor 830 that is arranged according to the previously presented figures. At least by this, the fluid path 812 extends through the plurality of microneedles 816, the fluid channel 814 and the metallised vias 821, 822. Thereby a fluid in the fluid path 812 may pass the sensor 830 while passing the first substrate 810 and the second substrate 820.

The microneedles 816 may for example be located at minimum distance from each other of 200 microns in order to avoid the effect of bed of nails.

The microneedles 816 may be oriented in a way that enables connection between at least a subset of microneedles using integrated, for instance etched, capillaries.

The microneedles 816 may all be combined with a capillary system enabling a capillary flow to a fluid exit port.

The second substrate may be wafer bonded or attached to the first substrate by other means, but may also be connected through capillary tubing or an equivalent flow system.

The sensor may also be configured for detecting a level of glucose in bodily fluid, i.e. a glucose sensor. Thereby a sensor for rapid and accurate detection of the level of glucose in bodily fluid may be provided.

The sensor may be configured for detecting a concentration or presence of lactate, carbon dioxide, or other molecules in bodily fluid. Thereby a sensor for rapid and accurate detection of the level of above mentioned molecule or other molecules, ions or biomarkers in bodily fluid may be provided.

While specific embodiments have been described, the skilled person will understand that various modifications and alterations are conceivable within the scope as defined in the appended claims.

## Claims

1. A sensor assembly (100, 200, 300, 400, 800) for sampling of a bodily fluid, comprising:
a first substrate (110, 210, 310, 410, 610, 810) comprising at least one capillary bore (111, 211, 311, 411, 511, 811) defining a fluid path (112, 212, 312, 412, 812) extending through said first substrate from a top side (113, 313, 413, 813) to a fluid channel (114, 214, 414, 814) on a bottom side (115, 215, 415, 815) of said first substrate;
a second substrate (120, 220, 320, 420, 620, 720, 820) arranged in connection with the first substrate, the fluid channel of the first substrate is in fluid communication with a first metallised via (121, 221, 421, 621, 721, 821) and a second metallised via (122, 222, 422, 622, 722, 822) formed in the second substrate, thereby extending the fluid path through the first metallised via and the second metallised via;
a sensor (130, 230, 430, 830) comprising a first electrode (231, 731) and a second electrode (232, 732) arranged on the second substrate in fluid communication with the fluid channel of the first substrate; wherein the first electrode is in electric contact with the first metallised via and wherein the second electrode is in electric contact with the second metallised via.

2. A sensor assembly according to claim 1, wherein the first substrate comprise a plurality of bores (211, 311, 811).

3. A sensor assembly according to any one preceding claim, further comprising:
means for applying sub-pressure to the fluid path to draw fluid from the capillary bores towards the second substrate.

4. A sensor assembly according to any one preceding claim, further comprising:
a plurality of microneedles (816) integrally formed on the first substrate, wherein each microneedle comprising:
an elongated body extending from a distal end thereof with a bevel to a proximal end thereof on the first substrate (810) along a longitudinal axis;
wherein the capillary bores (811) extend through the elongated body in a longitudinal direction thereof and further defining the fluid path (812);
wherein the proximal end is integrally formed with the first substrate and the fluid path is in fluid communication with the fluid channel of the first substrate.

5. A sensor assembly according to claim 4, wherein the second substrate (820) is arranged in connection with the first substrate (810) on a side opposite the plurality of microneedles.

6. A sensor assembly according to any one preceding claim, wherein the first electrode is shaped as a spiral and the second electrode is shaped as a spiral, and wherein the spiral shapes of the first and second electrodes are nested.

7. A sensor assembly according to any preceding claim, wherein the sensor is located on a side of the second substrate directed towards the first substrate.

8. A sensor assembly according to any one preceding claim, wherein the sensor is at least partly located on the side of the second substrate that is directed towards the first substrate.

9. A sensor assembly according to any one preceding claim, wherein the sensor is at least partly located in the first and second vias.

10. A sensor assembly according to any one preceding claim, wherein the vias are hollow,
thereby providing fluid communication between two opposite sides of the second substrate.

11. A sensor assembly according to any one preceding claim, wherein the sensor is an electrochemical sensor.

12. A sensor assembly according to any one preceding claim, wherein the wall surface of the vias are hydrophilic.

13. A measurement device comprising a sensor assembly according to any one of claims 1 to 12, further comprising a sub-pressurization device arranged in connection with the sensor assembly on a side opposite at least one microneedle and in fluid communication with the via of the second substrate, thereby providing sub-pressure through said via and the fluid channel.

## Patentansprüche

1. Sensoreinheit (100, 200, 300, 400, 800) zur Probenentnahme einer Körperflüssigkeit, Folgendes umfassend:
ein erstes Substrat (110, 210, 310, 410, 610, 810), das mindestens eine Kapillarbohrung (111, 211, 311, 411, 511, 811) umfasst, die einen Fluidweg (112, 212, 312, 412, 812) definiert, der sich durch das erste Substrat von einer Oberseite (113, 313, 413, 813) zu einem Fluidkanal (114, 214, 414, 814) auf einer Unterseite (115, 215, 415, 815) des ersten Substrats erstreckt;
ein zweites Substrat (120, 220, 320, 420, 620, 720, 820), das in Verbindung mit dem ersten Substrat angeordnet ist, wobei der Fluidkanal des ersten Substrats in Fluidkommunikation mit einer ersten metallisierten Durchkontaktierung (121, 221, 421, 621, 721, 821) und einer zweiten metallisierten Durchkontaktierung (122, 222, 422, 622, 722, 822), die in dem zweiten Substrat gebildet sind, steht, wodurch sich der Fluidweg durch die erste metallisierte Durchkontaktierung und die zweite metallisierte Durchkontaktierung erstreckt;
einen Sensor (130, 230, 430, 830), der eine erste Elektrode (231, 731) und eine zweite Elektrode (232, 732) umfasst, die auf dem zweiten Substrat in Fluidkommunikation mit dem Fluidkanal des ersten Substrats angeordnet sind; wobei die erste Elektrode in elektrischem Kontakt mit der ersten metallisierten Durchkontaktierung steht und wobei die zweite Elektrode in elektrischem Kontakt mit der zweiten metallisierten Durchkontaktierung steht.

2. Sensoreinheit nach Anspruch 1, wobei das erste Substrat eine Vielzahl von Bohrungen (211, 311, 811) umfasst.

3. Sensoreinheit nach einem der vorhergehenden Ansprüche, ferner Folgendes umfassend:
Mittel zum Anlegen von Unterdruck an den Fluidweg, um Fluid aus den Kapillarbohrungen in Richtung des zweiten Substrats zu ziehen.

4. Sensoreinheit nach einem der vorhergehenden Ansprüche, ferner Folgendes umfassend:
eine Vielzahl von Mikronadeln (816), die einstückig auf dem ersten Substrat gebildet sind, wobei jede Mikronadel Folgendes umfasst:
einen länglichen Körper, der sich von einem distalen Ende davon mit einer Abschrägung zu einem proximalen Ende davon auf dem ersten Substrat (810) entlang einer Längsachse erstreckt;
wobei sich die Kapillarbohrungen (811) durch den länglichen Körper in einer Längsrichtung davon erstrecken und ferner den Fluidweg (812) definieren;
wobei das proximale Ende einstückig mit dem ersten Substrat gebildet ist und der Fluidweg in Fluidkommunikation mit dem Fluidkanal des ersten Substrats steht.

5. Sensoreinheit nach Anspruch 4, wobei das zweite Substrat (820) in Verbindung mit dem ersten Substrat (810) auf einer der Vielzahl von Mikronadeln gegenüberliegenden Seite angeordnet ist.

6. Sensoreinheit nach einem der vorhergehenden Ansprüche, wobei die erste Elektrode als Spirale geformt ist und die zweite Elektrode als Spirale geformt ist und wobei die Spiralformen der ersten und der zweiten Elektrode ineinander verschachtelt sind.

7. Sensoreinheit nach einem der vorhergehenden Ansprüche, wobei sich der Sensor auf einer dem ersten Substrat zugewandten Seite des zweiten Substrats befindet.

8. Sensoreinheit nach einem der vorhergehenden Ansprüche, wobei sich der Sensor mindestens teilweise auf der dem ersten Substrat zugewandten Seite des zweiten Substrats befindet.

9. Sensoreinheit nach einem der vorhergehenden Ansprüche, wobei sich der Sensor mindestens teilweise in der ersten und der zweiten Durchkontaktierung befindet.

10. Sensoreinheit nach einem der vorhergehenden Ansprüche, wobei die Durchkontaktierungen hohl sind, wodurch sie eine Fluidkommunikation zwischen zwei gegenüberliegenden Seiten des zweiten Substrats bereitstellen.

11. Sensoreinheit nach einem der vorhergehenden Ansprüche, wobei der Sensor ein elektrochemischer Sensor ist.

12. Sensoreinheit nach einem der vorhergehenden Ansprüche, wobei die Wandoberfläche der Durchkontaktierungen hydrophil ist.

13. Messvorrichtung, umfassend eine Sensoreinheit nach einem der Ansprüche 1 bis 12, ferner umfassend eine Unterdruckbeaufschlagungsvorrichtung, die in Verbindung mit der Sensoreinheit auf einer mindestens einer Mikronadel gegenüberliegenden Seite und in Fluidverbindung mit der Durchkontaktierung des zweiten Substrats angeordnet ist, wodurch sie durch die Durchkontaktierung und den Fluidkanal Unterdruck bereitstellt.

## Revendications

1. Ensemble capteur (100, 200, 300, 400, 800) pour le prélèvement d'un liquide corporel, comprenant :
un premier substrat (110, 210, 310, 410, 610, 810) comprenant au moins un alésage capillaire (111, 211, 311, 411, 511, 811) définissant un trajet de fluide (112, 212, 312, 412, 812) s'étendant à travers ledit premier substrat depuis un côté supérieur (113, 313, 413, 813) jusqu'à un canal de fluide (114, 214, 414, 814) sur un côté inférieur (115, 215, 415, 815) dudit premier substrat ;
un second substrat (120, 220, 320, 420, 620, 720, 820) disposé en liaison avec le premier substrat, le canal de fluide du premier substrat étant en communication fluidique avec un premier trou d'interconnexion métallisé (121, 221, 421, 621, 721, 821) et un second trou d'interconnexion métallisé (122, 222, 422, 622, 722, 822) formés dans le second substrat, prolongeant ainsi le trajet de fluide à travers le premier trou d'interconnexion métallisé et le second trou d'interconnexion métallisé ;
un capteur (130, 230, 430, 830) comprenant une première électrode (231, 731) et une seconde électrode (232, 732) disposées sur le second substrat en communication fluidique avec le canal de fluide du premier substrat ; dans lequel la première électrode est en contact électrique avec le premier trou d'interconnexion métallisé et dans lequel la seconde électrode est en contact électrique avec le second trou d'interconnexion métallisé.

2. Ensemble capteur selon la revendication 1, dans lequel le premier substrat comprend une pluralité d'alésages (211, 311, 811).

3. Ensemble capteur selon l'une quelconque des revendications précédentes, comprenant en outre :
des moyens pour appliquer une sous-pression au trajet de fluide pour aspirer le fluide des alésages capillaires vers le second substrat.

4. Ensemble capteur selon l'une quelconque des revendications précédentes, comprenant en outre :
une pluralité de micro-aiguilles (816) formées d'un seul tenant sur le premier substrat, chaque micro-aiguille comprenant :
un corps allongé s'étendant à partir d'une extrémité distale de celui-ci avec un biseau à une extrémité proximale de celui-ci sur le premier substrat (810) le long d'un axe longitudinal ;
dans lequel les alésages capillaires (811) s'étendent à travers le corps allongé dans une direction longitudinale de celui-ci et définissent en outre le trajet de fluide (812) ;
dans lequel l'extrémité proximale est formée d'un seul tenant avec le premier substrat et le trajet de fluide est en communication fluidique avec le canal de fluide du premier substrat.

5. Ensemble capteur selon la revendication 4, dans lequel le second substrat (820) est disposé en liaison avec le premier substrat (810) sur un côté opposé à la pluralité de micro-aiguilles.

6. Ensemble capteur selon l'une quelconque des revendications précédentes, dans lequel la première électrode est en forme de spirale et la seconde électrode est en forme de spirale, et dans lequel les formes en spirale des première et seconde électrodes sont imbriquées.

7. Ensemble capteur selon l'une quelconque des revendications précédentes, dans lequel le capteur est situé sur un côté du second substrat dirigé vers le premier substrat.

8. Ensemble capteur selon l'une quelconque des revendications précédentes, dans lequel le capteur est au moins en partie situé sur le côté du second substrat qui est dirigé vers le premier substrat.

9. Ensemble capteur selon l'une quelconque des revendications précédentes, dans lequel le capteur est au moins en partie situé dans les premier et second trous d'interconnexion.

10. Ensemble capteur selon l'une quelconque des revendications précédentes, dans lequel les trous d'interconnexion sont creux, ce qui permet une communication fluidique entre deux côtés opposés du second substrat.

11. Ensemble capteur selon l'une quelconque des revendications précédentes, dans lequel le capteur est un capteur électrochimique.

12. Ensemble capteur selon l'une quelconque des revendications précédentes, dans lequel la surface de paroi des trous d'interconnexion est hydrophile.

13. Dispositif de mesure comprenant un ensemble capteur selon l'une quelconque des revendications 1 à 12, comprenant en outre un dispositif de sous-pression disposé en liaison avec l'ensemble capteur sur un côté opposé à au moins une micro-aiguille et en communication fluidique avec le trou d'interconnexion du second substrat, ce qui permet de fournir une sous-pression à travers ledit trou d'interconnexion et le canal fluidique.
